(19) Europäisches Patentamt
European Patent Office
Office européen des brevets

(11) **EP 4 450 104 B1**

(12) **EUROPÄISCHE PATENTSCHRIFT**

(45) Veröffentlichungstag und Bekanntmachung des Hinweises auf die Patenterteilung:
**12.11.2025 Patentblatt 2025/46**

(21) Anmeldenummer: **24167362.3**

(22) Anmeldetag: **28.03.2024**

(51) Internationale Patentklassifikation (IPC):
**A61M 16/00** (2006.01)   **A61M 16/10** (2006.01)
**A61M 16/08** (2006.01)

(52) Gemeinsame Patentklassifikation (CPC):
**A61M 16/1005; A61M 16/026; A61M 16/085;**
A61M 16/0891; A61M 2016/0027; A61M 2016/103

(54) **BESTIMMEN EINER GASKONZENTRATION IN EINEM BEATMUNGSSYSTEM**

DETERMINING A GAS CONCENTRATION IN A BREATHING SYSTEM

DÉTERMINATION D'UNE CONCENTRATION DE GAZ DANS UN SYSTÈME RESPIRATOIRE

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorität: **18.04.2023 DE 102023109772**

(43) Veröffentlichungstag der Anmeldung:
**23.10.2024 Patentblatt 2024/43**

(73) Patentinhaber: **Löwenstein Medical Technology
S.A.
2557 Luxembourg (LU)**

(72) Erfinder: **Borm, Petrus Johannes Josephus
5627 HP Eindhoven (NL)**

(74) Vertreter: **Löwenstein Medical IP
Löwenstein Medical Technology
GmbH + Co.KG IP Management
Kronsaalsweg 40
22525 Hamburg (DE)**

(56) Entgegenhaltungen:
US-A- 5 081 871       US-A- 6 000 397
US-A1- 2011 004 108    US-A1- 2021 109 084

## Beschreibung

### Technisches Gebiet

Die Erfindung betrifft ein Verfahren zum Bestimmen einer Gaskonzentration in einem Beatmungssystem. Des Weiteren betrifft die Erfindung eine Signalverarbeitungseinrichtung, ein Computerprogramm und ein computerlesbares Medium zum Ausführen des Verfahrens sowie ein Beatmungssystem.

### Stand der Technik

Die Konzentration von Kohlenstoffdioxid in einem Atemgas, das in einem Beatmungssystem strömt, kann durch eine Hauptstrommessung oder eine Nebenstrommessung bestimmt werden. Bei der Hauptstrommessung wird die Konzentration über einen im Atemweg, d. h. im Hauptstrom des Atemgases, angeordneten Gassensor direkt gemessen. Eine solche Messung ist in der Regel sehr genau. Allerdings benötigt der Gassensor für genaue Messergebnisse ein gewisses Mindestvolumen, was insbesondere bei der Beatmung von Neugeborenen oder Säuglingen zu einer unerwünschten Rückatmung von Kohlenstoffdioxid führen kann. Auch kann ein solcher Gassensor aufgrund seiner Größe und seines Gewichts für den Patienten unangenehm sein. Bei der Nebenstrommessung wird ein Teil des Atemgases an der jeweiligen Entnahmestelle, beispielsweise einer Nasenkanüle oder einer Maske, abgezweigt und zu einem externen Gassensor, der im Beatmungsgerät angeordnet sein kann, geführt. Eine solche Nebenstrommessung ist in der Regel weniger genau als die Hauptstrommessung.

US 2011/0004108 A1 offenbart ein System zur Überwachung der Herzleistung. Das System umfasst ein Beatmungsgerät, einen Gassensor und eine an den Gassensor angeschlossene Signalverarbeitungseinrichtung. Das Beatmungsgerät ist über eine Hauptleitung mit der Lunge eines Patienten verbindbar. Der Gassensor ist an eine von der Hauptleitung abzweigende Nebenleitung angeschlossen.

US 2021/0109084 A1 offenbart einen Nebenstromgasanalysator zum Messen einer Konzentration eines Atemgases anhand einer Gasprobe aus einer Gasprobenleitung mittels eines optischen Sensors, der Infrarotlicht durch eine Nebenstrommesskammer senden kann, und eines speziellen Farberkennungssystems.

US 5 081 871 A offenbart eine Probenahmevorrichtung mit einer Nebenstromöffnung zum Sammeln von Gas aus einem Nebenstrom. Um die Gaskonzentration in einem Hauptstrom zu schätzen, kann die aus dem Nebenstrom entnommene Masse des Gases bestimmt werden, die Masse durch das aus dem Nebenstrom entnommene Volumen des Gases dividiert werden und das Resultat durch einen empirisch bestimmten Prozentwert, der ein geschätztes Verhältnis der Gaskonzentration im Nebenstrom zur Gaskonzentration im Hauptstrom definiert, nämlich 71 %, dividiert werden.

US 6 000 397 A offenbart ein Beatmungsgerät, das über eine Inspirationsleitung, eine Exspirationsleitung und ein Y-Stück mit einem Patienten verbunden werden kann. Dabei ist ein erster Messschlauch an die Inspirationsleitung, ein zweiter Messschlauch an das Y-Stück und ein dritter Messschlauch an die Exspirationsleitung angeschlossen. Die Messschläuche können über ein Multiplexventil wahlweise mit einem Sauerstoffsensor, einem Kohlendioxidsensor und einem Druckmesser verbunden werden. Mittels einer Pumpe können Proben von Inspirations- und Exspirationsgasen durch die Messschläuche über das Multiplexventil zu den Gassensoren und dem Druckmesser gepumpt werden. Die Pumpe kann so gesteuert werden, dass - wenn eine Probe an den Gassensoren vorbeigepumpt wurde - die Pumprichtung umgekehrt und die Pumprate verringert wird, um die Probe in umgekehrter Richtung an den Gassensoren vorbeizupumpen.

### Offenbarung der Erfindung

Die Erfindung ist in den angehängten Ansprüchen definiert. Eine Aufgabe der Offenbarung kann darin gesehen werden, ein Verfahren zu schaffen, das eine möglichst genaue Bestimmung einer Gaskonzentration in einem Beatmungssystem ermöglicht und die vorgenannten Nachteile einer herkömmlichen Hauptstrommessung vermeidet. Eine weitere Aufgabe der Offenbarung kann darin gesehen werden, eine Signalverarbeitungseinrichtung, ein Computerprogramm und ein computerlesbares Medium zum Ausführen eines solchen Verfahrens sowie ein entsprechendes Beatmungssystem bereitzustellen.

Diese Aufgaben werden durch die Gegenstände der unabhängigen Ansprüche gelöst. Vorteilhafte Ausführungsformen der Offenbarung sind in den abhängigen Ansprüchen, der nachfolgenden Beschreibung und den beigefügten Figuren dargelegt.

Ein erster Aspekt der Offenbarung betrifft ein Verfahren zum Bestimmen einer Gaskonzentration in einem Beatmungssystem. Das Beatmungssystem umfasst: ein Beatmungsgerät mit einem Anschluss zum Bereitstellen eines Atemgases; eine Hauptleitung zum Verbinden des Anschlusses mit einer Patientenschnittstelle; eine Nebenleitung, die von der Hauptleitung abzweigt, sodass das Atemgas zumindest teilweise durch die Nebenleitung strömen kann; einen Gassensor zum Messen einer Gaskonzentration an mindestens einer ersten Stelle in der Nebenleitung; eine Signalverarbeitungseinrichtung. Das Verfahren umfasst die folgenden Schritte: Empfangen eines ersten Signals, das die durch den Gassensor gemessene Gaskonzentration anzeigt, in der Signalverarbeitungseinrichtung; Erzeugen eines zweiten Signals, das eine geschätzte Gaskonzentration an mindestens einer von der ersten Stelle abweichenden zweiten Stelle in der Hauptleitung anzeigt, aus dem ersten Signal durch die Signalverarbeitungseinrichtung.

Anders ausgedrückt kann die Gaskonzentration an einer bestimmten Stelle in der Nebenleitung, d. h. im Nebenstrom, direkt gemessen werden. Aus dem resultierenden Sensorsignal kann dann die Gaskonzentration an einer anderen Stelle in der Hauptleitung, d. h. im Hauptstrom, geschätzt werden, beispielsweise unter Verwendung eines mathematischen Modells des Gassensors und eines bekannten Volumenstroms des Atemgases in der Hauptleitung und/oder der Nebenleitung (siehe weiter unten).

Mit einer solchen Schätzung kann eine ähnliche Genauigkeit wie bei einer herkömmlichen Hauptstrommessung erreicht werden, ohne dass der Gassensor in die Hauptleitung integriert werden muss. Dadurch wird das Gesamtvolumen der Hauptleitung verringert. Dies kann die Wahrscheinlichkeit einer unerwünschten Rückatmung von ausgeatmetem Atemgas verringern. Zudem wird das Gesamtgewicht der Hauptleitung verringert. Dies kann den Komfort für den Patienten verbessern, insbesondere bei der Beatmung von Neugeborenen oder Säuglingen.

Das Verfahren kann computerimplementiert sein.

Unter "Signal" wie in "erstes Signal", "zweites Signal" oder "drittes Signal" (siehe weiter unten) kann ein analoges oder digitales Signal verstanden werden.

Ein zweiter Aspekt der Offenbarung betrifft eine Signalverarbeitungseinrichtung, die Mittel zum Ausführen des vor- und nachstehend beschriebenen Verfahrens umfasst.

Die Mittel können Hard- und/oder Softwaremodule umfassen. Insbesondere können die Mittel einen Prozessor umfassen, der konfiguriert ist, um das (computerimplementierte) Verfahren auszuführen. Zusätzlich können die Mittel einen Speicher und/oder eine Datenkommunikationsschnittstelle zur drahtlosen und/oder drahtgebundenen Datenkommunikation mit Peripheriegeräten umfassen. Alternativ kann die Signalverarbeitungseinrichtung ausschließlich als Hardware, beispielsweise in Form eines ASIC- oder FPGA-Bausteins, implementiert sein.

Es wird darauf hingewiesen, dass Merkmale des vor- und nachstehend beschriebenen Verfahrens auch Merkmale der Signalverarbeitungseinrichtung sein können (und umgekehrt).

Ein dritter Aspekt der Offenbarung betrifft ein Beatmungssystem. Das Beatmungssystem umfasst: ein Beatmungsgerät mit einem Anschluss zum Bereitstellen eines Atemgases; eine Hauptleitung zum Verbinden des Anschlusses mit einer Patientenschnittstelle; eine Nebenleitung, die von der Hauptleitung abzweigt, sodass das Atemgas zumindest teilweise durch die Nebenleitung strömen kann; einen Gassensor zum Messen einer Gaskonzentration an mindestens einer ersten Stelle in der Nebenleitung; eine Signalverarbeitungseinrichtung wie vor- und nachstehend beschrieben.

Unter "Beatmungsgerät" kann beispielsweise ein Heimbeatmungsgerät, ein Schlafatemtherapiegerät, ein Intensivbeatmungsgerät, ein Anästhesiegerät oder eine Kombination aus mindestens zwei dieser Beispiele verstanden werden. Das Beatmungsgerät kann zur invasiven und/oder nicht invasiven Beatmung ausgebildet sein.

Unter "Leitung" wie in "Hauptleitung" oder "Nebenleitung" kann beispielsweise ein (flexibler) Schlauch verstanden werden. Möglich sind aber auch starre Leitungen.

Das durch die Hauptleitung strömende Atemgas kann auch als Hauptstrom bezeichnet werden. Das durch die Nebenleitung strömende Atemgas kann auch als Nebenstrom bezeichnet werden.

Unter "Patientenschnittstelle" kann beispielsweise ein Intubationsschlauch, eine Nasenkanüle oder eine Atemmaske verstanden werden.

Die Nebenleitung kann sich beispielsweise in ihrem Gesamtvolumen und/oder ihrer Gesamtlänge signifikant von der Hauptleitung unterscheiden.

Unter "Gassensor" kann beispielsweise ein infrarotoptischer Sensor, auch nicht dispersiver Infrarotsensor oder kurz NDIR-Sensor genannt, oder ein Metalloxid-Halbleitersensor, kurz MOX-Sensor genannt, verstanden werden. Möglich ist auch ein Gassensor, der die Gaskonzentration anhand der Wärmeleitfähigkeit des jeweiligen Gases oder Gasgemisches, auch Wärmeleitfähigkeitsdetektor genannt, oder paramagnetisch misst. Der Gassensor kann zumindest teilweise, beispielsweise mit seiner aktiven Oberfläche, in der Nebenleitung angeordnet sein, um die Gaskonzentration direkt in der Nebenleitung zu messen.

Weitere Aspekte der Offenbarung betreffen ein Computerprogramm und ein computerlesbares Medium, auf dem das Computerprogramm gespeichert ist.

Das Computerprogramm umfasst Befehle, die einen Prozessor (beispielsweise einen Prozessor der vor- und nachstehend beschriebenen Signalverarbeitungseinrichtung) beim Ausführen des Computerprogramms durch den Prozessor veranlassen, das vor- und nachstehend beschriebene Verfahren auszuführen.

Das computerlesbare Medium kann ein flüchtiger oder nicht flüchtiger Datenspeicher sein. Beispielsweise kann das computerlesbare Medium eine Festplatte, ein USB-Speichergerät (*universal serial bus*), ein RAM (*random-access memory*), ein ROM (*read-only memory*), ein EPROM (*erasable programmable read-only memory*), ein EEPROM (*electrically erasable programmable read-only memory*), ein Flash-Speicher oder eine Kombination aus mindestens zwei dieser Beispiele sein. Das computerlesbare Medium kann auch ein Datenkommunikationsnetzwerk, das das Herunterladen von Programmcode ermöglicht (z. B. über das Internet), oder eine Cloud sein.

Es wird darauf hingewiesen, dass Merkmale des vor- und nachstehend beschriebenen Verfahrens auch Merkmale des Computerprogramms und/oder des computerlesbaren Mediums sein können (und umgekehrt).

Im Folgenden werden verschiedene Ausführungsformen der Offenbarung beschrieben. Diese Ausführungsformen sind nicht als Beschränkung des Umfangs der Offenbarung zu verstehen. Gemäß einer Ausführungsform kann die geschätzte Gaskonzentration abhängig von einer zeitlichen Ableitung der gemessenen Gaskonzentration bestimmt werden. Anders ausgedrückt kann zumindest ein Teil des zweiten Signals mit einer zeitlichen Ableitung des ersten Signals übereinstimmen oder darauf basieren. Auf diese Weise kann die Gaskonzentration an der zweiten Stelle oder den zweiten Stellen ausreichend genau geschätzt werden. Eine solche Schätzung hat zudem den Vorteil, dass sie sehr einfach als Hard- und/oder Software implementiert werden kann.

Gemäß einer Ausführungsform kann ein Zusammenhang zwischen der gemessenen Gaskonzentration und der geschätzten Gaskonzentration durch folgende Gleichung definiert sein:

$$\frac{d}{dt}(ConcSensor) = \frac{1}{\tau}(ConcAirway - ConcSensor).$$

Dabei steht "ConcSensor" für die gemessene Gaskonzentration, "ConcAirway" für die geschätzte Gaskonzentration und $\tau$ für eine Konstante, beispielsweise eine Zeitkonstante.

Anders ausgedrückt ist es möglich, dass das zweite Signal unter Verwendung eines (Umkehr-)Filters erster Ordnung aus dem ersten Signal erzeugt wird.

Die Gleichung kann beispielsweise durch Laplace-Transformation transformiert und/oder diskretisiert, d. h. digitalisiert, werden, um das zweite Signal zu erzeugen, d. h. Werte für die geschätzte Gaskonzentration zu berechnen. Die Gleichung kann beispielsweise als mathematische Funktion oder Lookup-Tabelle in einem Speicher der Signalverarbeitungseinrichtung hinterlegt sein.

Gemäß einer Ausführungsform kann die mindestens eine zweite Stelle eine Abzweigstelle, an der die Nebenleitung von der Hauptleitung abzweigt, umfassen. Die Abzweigstelle kann beispielsweise eine Stelle sein, an der bei einer herkömmlichen Hauptstrommessung die Gaskonzentration gemessen (statt wie hier geschätzt) würde. Anders ausgedrückt kann das erste Signal ausgewertet werden, um die Gaskonzentration zumindest an der Abzweigstelle zu schätzen. Dies ermöglicht beispielsweise eine sehr genaue Bestimmung der Menge an ausgeatmetem Kohlenstoffdioxid.

Gemäß einer Ausführungsform kann das zweite Signal unter Verwendung eines mathematischen Modells des Gassensors erzeugt werden. Das mathematische Modell kann beispielsweise ein System von Differentialgleichungen zum Definieren eines Zusammenhangs zwischen dem ersten und dem zweiten Signal umfassen. Das mathematische Modell kann durch bestimmte Parameter, die physikalische und/oder chemische Eigenschaften des Gassensors beschreiben, definiert sein. Das mathematische Modell kann verwendet werden, um näherungsweise ein Signal des Gassensors zu simulieren, das der Gassensor erzeugen würde, wenn er die Gaskonzentration statt an der ersten Stelle oder den ersten Stellen an der zweiten Stelle oder den zweiten Stellen messen würde. Das mathematische Modell kann beispielsweise durch mindestens einen der folgenden Parameter definiert sein: eine oder mehrere Zeitkonstanten, ein Volumen der Haupt- und/oder der Nebenleitung, ein Volumenstrom des Atemgases in der Haupt- und/oder der Nebenleitung, ein Totvolumen. Die Zeitkonstante kann beispielsweise ein Quotient aus dem Volumen und dem Volumenstrom sein. Das mathematische Modell kann beispielsweise durch Analyse einer Sprungantwort, die aus einer sprunghaften Änderung der Gaskonzentration an der zweiten Stelle oder den zweiten Stellen resultiert, bestimmt worden sein.

Gemäß einer Ausführungsform kann das zweite Signal abhängig von einem bekannten Messvolumen des Gassensors erzeugt werden. Unter "Messvolumen" kann das Volumen einer vom Atemgas durchströmten Messkammer des Gassensors verstanden werden. Das bekannte Messvolumen kann beispielsweise ein Parameter des mathematischen Modells das Gassensors sein.

Gemäß einer Ausführungsform kann das zweite Signal abhängig von einem bekannten Volumenstrom des Atemgases in der Hauptleitung erzeugt werden.

Gemäß einer Ausführungsform kann das zweite Signal abhängig von einem bekannten Volumenstrom des Atemgases in der Nebenleitung erzeugt werden.

Ein bekannter Volumenstrom, auch Fluss genannt, kann beispielsweise ein gemessener Volumenstrom sein und/oder durch die bekannten Eigenschaften der Hauptleitung und/oder der Nebenleitung vorgegeben sein. Der bekannte Volumenstrom des Atemgases in der Hauptleitung und/oder der Nebenleitung kann beispielsweise ein Parameter des mathematischen Modells das Gassensors sein.

Gemäß einer Ausführungsform kann der bekannte Volumenstrom des Atemgases in der Nebenleitung unabhängig von einem (bekannten oder unbekannten) Druck (und/oder Volumenstrom) des Atemgases in der Hauptleitung entweder einen konstanten Betrag oder eine konstante Richtung oder sowohl einen konstanten Betrag als auch eine konstante Richtung haben. Anders ausgedrückt kann der Volumenstrom in der Nebenleitung so gesteuert werden, dass er mehr oder weniger gleich bleibt, und zwar auch dann, wenn sich der Druck (und/oder der Volumenstrom), etwa beim Übergang zwischen Ein- und Ausatmung, in der Hauptleitung oder in einem Teilabschnitt der Hauptleitung signifikant ändert.

Gemäß einer Ausführungsform kann der bekannte Volumenstrom des Atemgases in der Nebenleitung 2 ml/s oder weniger, insbesondere 1 ml/s oder weniger, betragen. Solche Werte sind besonders günstig für die Beatmung von Neugeborenen oder Säuglingen.

Gemäß einer Ausführungsform kann $\tau$ = *VolumeSensor/Flow* sein. Dabei steht "VolumeSensor" für das bekannte Messvolumen des Gassensors und "Flow" für den bekannten Volumenstrom des Atemgases in der Nebenleitung. Anders ausgedrückt kann der Zusammenhang zwischen der gemessenen Gaskonzentration und der geschätzten Gaskonzentration durch folgende Gleichung definiert sein:

$$\frac{\mathrm{d}}{\mathrm{d}t}(ConcSensor) = Flow/VolumeSensor * (ConcAirway - ConcSensor).$$

Gemäß einer Ausführungsform kann die gemessene Gaskonzentration eine Kohlenstoffdioxidkonzentration sein.

Gemäß einer Ausführungsform kann die geschätzte Gaskonzentration eine Kohlenstoffdioxidkonzentration sein.

Gemäß einer Ausführungsform kann das Verfahren ferner einen Schritt des Filterns des zweiten Signals umfassen, um das zweite Signal zu glätten. Beispiele für geeignete Filter sind Bandpass-, Hochpass- oder Tiefpassfilter. Möglich sind aber auch andere Filter. Auf diese Weise kann beispielsweise unerwünschtes Rauschen im zweiten Signal unterdrückt werden. Somit kann die Genauigkeit bei der Bestimmung der Gaskonzentration weiter verbessert werden.

Gemäß einer Ausführungsform kann das zweite Signal mit einem Tiefpassfilter gefiltert werden. Damit kann unerwünschtes Rauschen im zweiten Signal mit einfachen Mitteln besonders wirkungsvoll unterdrückt werden.

Gemäß einer Ausführungsform kann ferner ein drittes Signal, das eine durch einen Drucksensor gemessene Druckdifferenz zwischen verschiedenen Stellen der Hauptleitung anzeigt, in der Signalverarbeitungseinrichtung empfangen werden. Das zweite Signal kann dann unter zusätzlicher Verwendung des dritten Signals erzeugt werden. Das dritte Signal kann beispielsweise dazu verwendet werden, einen Volumenstrom des Atemgases in der Hauptleitung und/oder der Nebenleitung und/oder einen Beatmungsdruck zu bestimmen und/oder eine Pumpe des Beatmungssystems (siehe weiter unten) so anzusteuern, dass sich ein Volumenstrom des Atemgases in der Hauptleitung und/oder der Nebenleitung einem bestimmten Sollwert annähert. In Bezug auf den Volumenstrom des Atemgases in der Nebenleitung kann der Sollwert beispielsweise 2 ml/s oder weniger, insbesondere 1 ml/s oder weniger, betragen.

Gemäß einer Ausführungsform kann das Beatmungssystem ferner einen Atemgasfilter zum Filtern des Atemgases, bevor es in den Gassensor eintritt, umfassen. Dadurch können Messfehler infolge von Verunreinigungen und/oder Beschädigungen des Gassensors vermieden werden.

Gemäß einer Ausführungsform kann der Atemgasfilter entweder eine Wasserfalle oder einen Partikelfilter oder sowohl eine Wasserfalle als auch einen Partikelfilter umfassen. Unter "Partikelfilter" kann vor- und nachstehend insbesondere ein hydrophober Partikelfilter verstanden werden, der zusätzlich Feuchtigkeit aus dem Atemgas entfernen kann.

Gemäß einer Ausführungsform kann das Beatmungssystem, insbesondere die Nebenleitung, so ausgebildet sein, dass ein Volumenstrom des Atemgases in der Nebenleitung unabhängig von einem Druck (und/oder einem Volumenstrom) des Atemgases in der Hauptleitung entweder einen konstanten Betrag oder eine konstante Richtung oder sowohl einen konstanten Betrag als auch eine konstante Richtung hat. Dadurch kann sichergestellt werden, dass die Messung der Gaskonzentration zu verschiedenen Zeitpunkten unter den annähernd gleichen Messbedingungen erfolgt.

Gemäß einer Ausführungsform kann das Beatmungssystem, insbesondere die Nebenleitung, so ausgebildet sein, dass ein Volumenstrom des Atemgases in der Nebenleitung 2 ml/s oder weniger, insbesondere 1 ml/s oder weniger, beträgt. Solche Werte sind besonders günstig für die Beatmung von Neugeborenen oder Säuglingen.

Gemäß einer Ausführungsform kann die Nebenleitung eine Blende zum Einstellen des Volumenstroms des Atemgases in der Nebenleitung umfassen. Unter "Blende" kann eine speziell geformte, insbesondere sprungartige lokale Verengung des Querschnitts der Nebenleitung verstanden werden, beispielsweise in Form einer Scheibe oder eines Lochgitters, wobei die lokale Verengung einen Strömungswiderstand bildet. Die Blende kann beispielsweise eine Öffnung mit einem in Strömungsrichtung variierenden Durchmesser haben. Mit einer solchen Blende konnten in Versuchen sehr gute Ergebnisse erzielt werden. Alternativ kann auch eine Drossel oder ein Steuerventil verwendet werden.

Gemäß einer Ausführungsform kann die Nebenleitung einen ersten Leitungsabschnitt und einen zweiten Leitungsabschnitt umfassen. In diesem Fall kann der Gassensor im ersten Leitungsabschnitt angeordnet sein und der zweite Leitungsabschnitt kann den Gassensor überbrücken. Eine solche Überbrückung ermöglicht es, den Volumenstrom des Atemgases durch die Nebenleitung auch dann aufrechtzuerhalten, wenn der Weg über den Gassensor aus irgendeinem Grund blockiert oder verengt ist.

Gemäß einer Ausführungsform kann der erste Leitungsabschnitt eine erste Blende zum Einstellen eines Volumenstroms des Atemgases im ersten Leitungsabschnitt umfassen. Auf diese Weise kann der Volumenstrom im ersten Leitungsabschnitt mit geringem konstruktivem Aufwand auf einen bestimmten Wert eingestellt werden.

Gemäß einer Ausführungsform kann der zweite Leitungsabschnitt eine zweite Blende zum Einstellen eines Volumenstroms des Atemgases im zweiten Leitungsabschnitt umfassen. Auf diese Weise kann der Volumenstrom im zweiten Leitungsabschnitt mit geringem konstruktivem Aufwand auf einen bestimmten Wert eingestellt werden.

Durch die Kombination der beiden Blenden kann sichergestellt werden, dass sich der Volumenstrom des Atemgases - insbesondere dort, wo es in den Gassensor eintritt - einem bestimmten Sollwert annähert. Beispielsweise kann die zweite Blende eine kleinere Öffnung als die erste Blende haben. Dies hat den Effekt, dass im zweiten Leitungsabschnitt ein ausreichend starker Volumenstrom erzeugt wird, falls der erste Leitungsabschnitt aus irgendeinem Grund blockiert ist, insbesondere falls ein dem Gassensor vorgeschalteter Atemgasfilter im ersten Leitungsabschnitt (nachstehend auch erster Atemgasfilter genannt) verstopft ist, sodass der verstopfte Atemgasfilter entsprechend gespült und wieder durchgängig gemacht werden kann.

Gemäß einer Ausführungsform kann die erste Blende zwischen einer Austrittsöffnung des Gassensors und einer Mündungsstelle, an der der zweite Leitungsabschnitt in den ersten Leitungsabschnitt mündet, angeordnet sein.

Gemäß einer Ausführungsform kann der erste Leitungsabschnitt einen ersten Atemgasfilter umfassen. Der erste Atemgasfilter kann beispielsweise einen Partikelfilter und/oder eine Wasserfalle umfassen. Der erste Atemgasfilter kann so angeordnet sein, dass er durch den Volumenstrom des Atemgases im zweiten Leitungsabschnitt gespült wird. Auf diese Weise kann beispielsweise Schleim oder Feuchtigkeit aus dem ersten Atemgasfilter über den zweiten Leitungsabschnitt entfernt werden. Somit können Verstopfungen des ersten Atemgasfilters vermieden oder beseitigt werden.

Gemäß einer Ausführungsform kann der zweite Leitungsabschnitt einen zweiten Atemgasfilter umfassen. Der zweite Atemgasfilter kann beispielsweise einen Partikelfilter und/oder eine Wasserfalle umfassen.

Der erste und der zweite Atemgasfilter können vom gleichen Typ oder von unterschiedlichen Typen sein.

Gemäß einer Ausführungsform kann ein Auslass des ersten Atemgasfilters mit einer Eintrittsöffnung des Gassensors verbunden sein. Zusätzlich oder alternativ kann der Auslass mit einem Einlass des zweiten Atemgasfilters verbunden sein. Anders ausgedrückt kann der erste Atemgasfilter so mit dem Gassensor verbunden sein, dass der Gassensor die Gaskonzentration in dem vom ersten Atemgasfilter gefilterten Atemgas messen kann. Der erste und der zweite Atemgasfilter können miteinander und/oder mit mindestens einem dritten Atemgasfilter in Reihe geschaltet sein, um eine zwei- oder mehrstufige Filterung des Atemgases zu ermöglichen.

Gemäß einer Ausführungsform kann der Auslass des ersten Atemgasfilters über mindestens einen dritten Atemgasfilter mit dem Einlass des zweiten Atemgasfilters verbunden sein. Anders ausgedrückt können mindestens drei Atemgasfilter miteinander in Reihe geschaltet sein, um das Atemgas zu filtern. Jeder Atemgasfilter kann beispielsweise einen Partikelfilter und/oder eine Wasserfalle umfassen.

Gemäß einer Ausführungsform kann der erste Atemgasfilter einen ersten Partikelfilter umfassen, der zweite Atemgasfilter einen zweiten Partikelfilter umfassen und der mindestens eine dritte Atemgasfilter eine Wasserfalle umfassen. Dies ermöglicht eine besonders gründliche Filterung des Atemgases.

Gemäß einer Ausführungsform kann das Beatmungssystem eine in der Haupt- oder der Nebenleitung angeordnete Pumpe zum Fördern des Atemgases umfassen. Alternativ kann mindestens eine erste Pumpe in der Hauptleitung und mindestens eine zweite Pumpe in der Nebenleitung angeordnet sein. Die Pumpe kann stattdessen auch im Beatmungsgerät angeordnet sein. Die Pumpe kann beispielsweise ausgebildet sein, um einen Unterdruck in der Haupt- und/oder der Nebenleitung zu erzeugen. Beispielsweise kann der Unterdruck um einen Faktor zwischen 0,4 und 0,6, insbesondere zwischen 0,50 und 0,55, vom Umgebungsdruck abweichen. Es ist möglich, dass die Pumpe unter Verwendung des ersten Signals, des zweiten Signals oder des dritten Signals oder unter Verwendung von mindestens zwei dieser Signale angesteuert wird. Beispielsweise kann anhand des dritten Signals eine Blockade oder eine sonstige unerwünschte Querschnittsverengung der Hauptleitung erkannt werden. Als Reaktion darauf kann die Pumpe so angesteuert werden, dass in den Alveolen des beatmeten Patienten, insbesondere eines Neugeborenen oder Säuglings, kein negativer Druck entsteht. Beispielsweise kann die Pumpe bei der Beatmung eines Neugeborenen oder Säuglings deaktiviert werden, wenn der gemessene Druck in der Hauptleitung eine bestimmte Schwelle überschreitet, beispielsweise unter 2 mbar fällt. Aus dem dritten Signal kann zusätzlich oder alternativ ein aktueller (positiver oder negativer) Wert eines Beatmungsdrucks bestimmt werden, mit dem beispielsweise die Pumpe so angesteuert werden kann, dass sich der aktuelle Wert einem bestimmten Sollwert annähert.

Gemäß einer Ausführungsform kann die Pumpe im ersten Leitungsabschnitt angeordnet sein und der zweite Leitungsabschnitt zwischen der Pumpe und dem Gassensor in den ersten Leitungsabschnitt münden.

Gemäß einer Ausführungsform kann das Beatmungssystem eine in der Haupt- oder der Nebenleitung angeordnete Absaugeinrichtung zum Absaugen von Flüssigkeit, insbesondere von Sekret oder Schleim, umfassen. Somit können Verstopfungen vermieden werden.

Gemäß einer Ausführungsform kann die Absaugeinrichtung in der Hauptleitung zwischen einer Abzweigstelle, an der die Nebenleitung von der Hauptleitung abzweigt, und der Patientenschnittstelle angeordnet sein. Auf diese Weise kann vermieden werden, dass Flüssigkeit, insbesondere Sekret oder Schleim, beim Ausatmen in die Haupt- und die Nebenleitung eindringt.

Alternativ kann die Absaugeinrichtung in der Hauptleitung zwischen dem Anschluss des Beatmungsgeräts und einer Abzweigstelle, an der die Nebenleitung von der Hauptleitung abzweigt, angeordnet sein. Beispielsweise kann die Absaugeinrichtung dabei zwischen einer Stelle, an der ein Drucksensor mit der Hauptleitung verbunden ist, und der Abzweigstelle angeordnet sein.

Gemäß einer Ausführungsform kann das Beatmungssystem einen Drucksensor zum Messen einer Druckdifferenz zwischen verschiedenen Stellen der Hauptleitung umfassen. Der Drucksensor kann beispielsweise mit der Signalverarbeitungseinrichtung zur Datenkommunikation verbunden sein.

Gemäß einer Ausführungsform kann das Beatmungssystem einen zusätzlichen Drucksensor zum Messen eines Beatmungsdrucks, mit dem der Patient aktuell beatmet wird, umfassen, beispielsweise an der Abzweigstelle oder an einer sonstigen Stelle möglichst nah an der Patientenschnittstelle.

Gemäß einer Ausführungsform kann ein erster Druckanschluss des Drucksensors zwischen dem Anschluss des Beatmungsgeräts und einer Abzweigstelle, an der die Nebenleitung von der Hauptleitung abzweigt, mit der Hauptleitung verbunden sein. Ein zweiter Druckanschluss des Drucksensors kann zwischen einer Stelle, an der der erste Druckanschluss mit der Hauptleitung verbunden ist, und der Patientenschnittstelle mit der Hauptleitung verbunden sein.

Gemäß einer Ausführungsform kann der zweite Druckanschluss zwischen dem Anschluss des Beatmungsgeräts und der Abzweigstelle mit der Hauptleitung verbunden sein.

Gemäß einer Ausführungsform kann der zweite Druckanschluss an der Abzweigstelle mit der Hauptleitung verbunden sein.

Gemäß einer Ausführungsform kann die Nebenleitung eine Öffnung haben, aus der das Atemgas in die Umgebung strömen kann. Beispielsweise kann die Öffnung durch ein freies, offenes Ende der Nebenleitung gebildet sein, sodass das Atemgas, nachdem es den Gassensor passiert hat, in die Umgebung strömen kann.

Gemäß einer Ausführungsform kann das Beatmungsgerät einen weiteren Anschluss zum Anschließen der Nebenleitung umfassen, sodass das Atemgas aus der Nebenleitung zurück ins Beatmungsgerät strömen kann. Genauer gesagt kann das Atemgas, nachdem es den Gassensor passiert hat, über den weiteren Anschluss wieder zurück ins Beatmungsgerät strömen. Somit kann das Beatmungsgerät das zurückgeströmte Atemgas, beispielsweise nach einer entsprechenden Aufbereitung im Beatmungsgerät, wieder an dem mit der Hauptleitung verbundenen Anschluss bereitstellen. Dies kann die Effizienz des Beatmungssystems verbessern.

Gemäß einer Ausführungsform kann das Beatmungssystem zur Beatmung von Neugeborenen und/oder Säuglingen ausgebildet sein.

**Kurze Beschreibung der Zeichnungen**

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.

Fig. 1 zeigt ein Beatmungssystem gemäß einer Ausführungsform der Erfindung.
Fig. 2 zeigt ein Diagramm zur Veranschaulichung eines Signals, wie es in einem Verfahren gemäß einer Ausführungsform der Offenbarung erzeugt wird.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

**Ausführungsformen der** Offenbarung

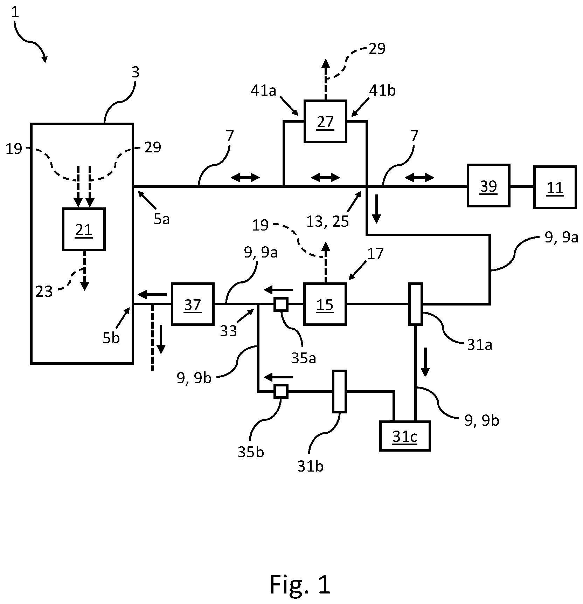

Fig. 1 zeigt ein Beatmungssystem 1, das ein Beatmungsgerät 3 mit einem Anschluss 5a zum Bereitstellen eines Atemgases, eine Hauptleitung 7 und eine Nebenleitung 9 umfasst. Die Hauptleitung 7 verbindet den Anschluss 5a mit einer Patientenschnittstelle 11, beispielsweise einem Intubationsschlauch, einer Nasenkanüle oder einer Atemmaske. Die Nebenleitung 9 zweigt an einer Abzweigstelle 13 von der Hauptleitung 7 ab, sodass das Atemgas bei der Beatmung nicht nur zwischen dem Anschluss 5a und der Patientenschnittstelle 11, sondern teilweise auch durch die Nebenleitung 9 strömen kann. Mögliche Flussrichtungen des Atemgases sind in Fig. 1 mit durchgehenden Pfeilen markiert.

Das Beatmungssystem 1 umfasst ferner einen Gassensor 15 zum Messen einer Gaskonzentration an mindestens einer ersten Stelle 17 in der Nebenleitung 9. Der Gassensor 15, beispielsweise ein Kohlenstoffdioxidsensor, kann zumindest teilweise in der Nebenleitung 9 angeordnet sein. Der Gassensor 15 ist ausgebildet, um ein erstes Signal 19 zu erzeugen, das die an der ersten Stelle 17 oder den ersten Stellen 17 gemessene Gaskonzentration anzeigt. Der Gassensor 15 ist mit einer Signalverarbeitungseinrichtung 21 des Beatmungssystems 1 zur Datenkommunikation verbunden. Die Signalverarbeitungseinrichtung 21 kann, wie hier gezeigt, eine Komponente des Beatmungsgeräts 3 sein.

Die Signalverarbeitungseinrichtung 21 ist ausgebildet, um ein Verfahren zum Bestimmen einer Gaskonzentration im Beatmungssystem 1 auszuführen. Dazu kann die Signalverarbeitungseinrichtung 21 beispielsweise einen Speicher und einen Prozessor (nicht gezeigt) umfassen. Im Speicher kann ein Computerprogramm gespeichert sein und der Prozessor kann konfiguriert sein, um das Verfahren durch Ausführen des Computerprogramms auszuführen.

Das Verfahren umfasst einen ersten Schritt, in dem das erste Signal 19 in der Signalverarbeitungseinrichtung 21 über eine geeignete Datenkommunikationsverbindung, die drahtgebunden oder drahtlos sein kann, empfangen wird. In einem zweiten Schritt erzeugt die Signalverarbeitungseinrichtung 21 aus dem ersten Signal 19 ein zweites Signal 23, das eine geschätzte Gaskonzentration an mindestens einer von der ersten Stelle 17 abweichenden zweiten Stelle 25 in der Hauptleitung 7 anzeigt. In diesem Beispiel stimmt die zweite Stelle 25 mit der Abzweigstelle 13 überein. Die zweite Stelle 25 kann aber auch eine andere Stelle der Hauptleitung 7 sein.

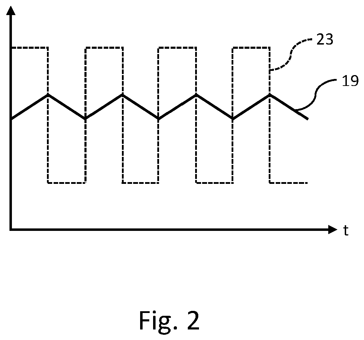

Im einfachsten Fall erzeugt die Signalverarbeitungseinrichtung 21 das zweite Signal 23 abhängig von einer zeitlichen Ableitung des ersten Signals 19, wie es beispielhaft in Fig. 2 gezeigt ist. Die Signale 19, 23 sind in Fig. 2 in idealisierter Form dargestellt. In der Realität können die Signale 19, 23 eine leicht abweichende Form haben, beispielsweise mehr oder weniger verrauscht sein.

Zusätzlich oder alternativ kann das zweite Signal 23 unter Verwendung eines mathematischen Modells des Gassensors 15 erzeugt werden.

Die geschätzte Gaskonzentration kann besonders genau bestimmt werden, indem das erste Signal 19, d. h. die gemessene Gaskonzentration, entsprechend der folgenden Gleichung verarbeitet wird:

$$\frac{\mathrm{d}}{\mathrm{d}t}(ConcSensor) = Flow/VolumeSensor * (ConcAirway - ConcSensor).$$

Dabei steht "ConcSensor" für die an der ersten Stelle 17 oder den ersten Stellen 17 gemessene Gaskonzentration, "Flow" für einen bekannten Volumenstrom des Atemgases in der Nebenleitung 9, "VolumeSensor" für ein bekanntes Messvolumen des Gassensors 15 und "ConcAirway" für die geschätzte Gaskonzentration.

Das Beatmungssystem 1, insbesondere die Hauptleitung 7 und/oder die Nebenleitung 9, kann so ausgebildet sein, dass der Volumenstrom in der Nebenleitung 9 annähernd konstant ist, d. h. den gleichen Betrag und die gleiche Richtung hat, auch wenn sich der Druck in der Hauptleitung 7 ändert, beispielsweise beim Übergang zwischen Ein- und Ausatmung. Insbesondere bei der Beatmung von Neugeborenen oder Säuglingen ist es günstig, wenn der Volumenstrom in der Nebenleitung 9 2 ml/s oder weniger oder sogar 1 ml/s oder weniger beträgt.

Zusätzlich kann das zweite Signal 23 durch die Signalverarbeitungseinrichtung 21 geglättet werden, beispielsweise unter Verwendung eines Tiefpassfilters, um unerwünschtes Rauschen zu unterdrücken.

Möglich ist auch, dass bei der Schätzung der Gaskonzentration an der zweiten Stelle 25 oder den zweiten Stellen 25 ein von einem Drucksensor 27 bereitgestelltes drittes Signal 29 berücksichtigt wird, wobei das dritte Signal 29 eine Druckdifferenz zwischen verschiedenen Stellen der Hauptleitung 7 anzeigt. Dies kann die Genauigkeit der Schätzung weiter verbessern. Beispielsweise kann anhand der (gemessenen) Druckdifferenz mindestens eine der folgenden Größen bestimmt werden: ein aktueller Volumenstrom zwischen dem Anschluss 5a und der Abzweigstelle 13, ein aktueller Volumenstrom zwischen der Abzweigstelle 13 und der Patientenschnittstelle 11, ein aktueller Volumenstrom in der Nebenleitung 7, ein aktueller Beatmungsdruck in der Hauptleitung 7, insbesondere an der Abzweigstelle 13.

Mithilfe des vorstehend beschriebenen Verfahrens kann die Gaskonzentration, hier die Kohlenstoffdioxidkonzentration, an einer oder mehreren bestimmten Stellen in der Hauptleitung 7 in Echtzeit bestimmt werden, ohne dass ein entsprechender Gassensor in der Hauptleitung 7, d. h. im Hauptstrom, platziert werden muss. Dies hat insbesondere bei der Beatmung von Neugeborenen und/oder Säuglingen den Vorteil, dass aufgrund des verringerten Volumens der Hauptleitung 7 kein oder deutlich weniger ausgeatmetes Atemgas zurückgeatmet wird. Außerdem wird die Hauptleitung 7 durch die Anordnung des Gassensors 15 in der Nebenleitung 9 leichter, was die Handhabung vereinfacht und den Komfort für den Patienten verbessert.

In dem in Fig. 1 gezeigten Beispiel umfasst die Nebenleitung 9 einen ersten Leitungsabschnitt 9a und einen zweiten Leitungsabschnitt 9b, wobei der Gassensor 15 im ersten Leitungsabschnitt 9a angeordnet ist und der zweite Leitungsabschnitt 9b den im ersten Leitungsabschnitt 9a angeordneten Gassensor 15 überbrückt.

Zwischen der Abzweigstelle 13 und einer Eintrittsöffnung des Gassensors 15, durch die das Atemgas in den Gassensor 15 eintritt, kann ein erster Atemgasfilter 31a, beispielsweise in Form eines ersten Partikelfilters, im ersten Leitungsabschnitt 9a angeordnet sein, sodass das Atemgas gefiltert wird, bevor es in den Gassensor 15 eintritt.

Zusätzlich oder alternativ kann im zweiten Leitungsabschnitt 9b ein zweiter Atemgasfilter 31b, beispielsweise in Form eines zweiten Partikelfilters, angeordnet sein, sodass das Atemgas gefiltert wird, bevor es den zweiten Leitungsabschnitt 9b verlässt.

Es ist möglich, dass zwischen den Atemgasfiltern 31a, 31b mindestens ein dritter Atemgasfilter 31c angeordnet ist, beispielsweise in Form einer Wasserfalle im zweiten Leitungsabschnitt 9b. Dies bewirkt, dass das aus dem ersten Atemgasfilter 31a austretende Atemgas zusätzlich gefiltert wird, bevor es in den zweiten Atemgasfilter 31b eintritt.

Um den Volumenstrom in der Nebenleitung 9 konstant zu halten, kann die Nebenleitung 9 eine oder mehrere Blenden haben.

In diesem Beispiel ist zwischen einer Austrittsöffnung des Gassensors 15, durch die das Atemgas aus dem Gassensor 15 austritt, und einer Mündungsstelle 33, an der der zweite Leitungsabschnitt 9b in den ersten Leitungsabschnitt 9a mündet, eine erste Blende 35a angeordnet, die den Volumenstrom im ersten Leitungsabschnitt 9a einstellt.

Zusätzlich kann im zweiten Leitungsabschnitt 9b, beispielsweise zwischen der Mündungsstelle 33 und einem Auslass des zweiten Atemgasfilters 31b, eine zweite Blende 35b angeordnet sein, die den Volumenstrom im zweiten Leitungsabschnitt 9b einstellt.

Genauer gesagt stellt die erste Blende 35a in diesem Fall nur einen Teil des Volumenstroms im ersten Leitungsabschnitt 9a ein. Der andere Teil wird durch die zweite Blende 35b eingestellt. Der (Gesamt-)Volumenstrom durch den ersten Leitungsabschnitt 9a entspricht somit einer Summe des Volumenstroms durch die erste Blende 35a und des Volumenstroms durch die zweite Blende 35b.

Die Blenden 35a, 35b können so ausgebildet sein, dass der Gesamtvolumenstrom in der Nebenleitung 9, insbesondere der Volumenstrom zwischen der Abzweigstelle 13 und der Eintrittsöffnung des Gassensors 15, 2 ml/s oder weniger oder sogar 1 ml/s oder weniger beträgt.

Zum Fördern des Atemgases kann eine geeignete Pumpe 37 in der Nebenleitung 9 angeordnet sein, beispielsweise im ersten Leitungsabschnitt 9a zwischen der Mündungsstelle 33 und einem Ende der Nebenleitung 9. Die Pumpe 37 kann alternativ in der Hauptleitung 7 oder im Beatmungsgerät 3 angeordnet sein.

Das Ende der Nebenleitung 9 kann offen sein, sodass das Atemgas in die Umgebung strömen kann, wie es in Fig. 1 mit einer gestrichelten Linie angedeutet ist.

Alternativ kann das Ende der Nebenleitung 9 mit einem weiteren Anschluss 5b des Beatmungsgeräts 3 verbunden sein, sodass das Atemgas aus der Nebenleitung 9 zurück ins Beatmungsgerät 3 strömen kann, um von dort, beispielsweise nach einer geeigneten Aufbereitung im Beatmungsgerät 3, wieder in die Hauptleitung 7 geleitet zu werden.

Um Verstopfungen oder sonstige Beeinträchtigungen bei der Beatmung infolge von Sekreten oder Kondenswasser zu vermeiden, kann das Beatmungssystem 1 eine geeignete Absaugeinrichtung 39 zum Absaugen und Auffangen solcher Flüssigkeiten umfassen. In diesem Beispiel ist die Absaugeinrichtung 39 in der Hauptleitung 7 zwischen der Abzweigstelle 13 und der Patientenschnittstelle 11 angeordnet. Auf diese Weise können unter anderem Verstopfungen des Drucksensors 27 vermieden werden.

Es ist möglich, dass ein erster Druckanschluss 41a des Drucksensors 27 zwischen der Absaugeinrichtung 39 und der Abzweigstelle 13 mit der Hauptleitung 7 verbunden ist und ein zweiter Druckanschluss 41b des Drucksensors 27 zwischen der Abzweigstelle 13 und der Patientenschnittstelle 11 mit der Hauptleitung 7 verbunden ist. Möglich ist auch eine Konfiguration, bei der beide Druckanschlüsse 41a, 41b zwischen dem Anschluss 5a und der Abzweigstelle 13 mit der Hauptleitung 7 verbunden sind.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

**Liste der Bezugszeichen**

| | |
|---|---|
| 1 | Beatmungssystem |
| 3 | Beatmungsgerät |
| 5a | Anschluss |
| 5b | weiterer Anschluss |
| 7 | Hauptleitung |
| 9 | Nebenleitung |
| 9a | erster Leitungsabschnitt |
| 9b | zweiter Leitungsabschnitt |
| 11 | Patientenschnittstelle |
| 13 | Abzweigstelle |
| 15 | Gassensor |

17 erste Stelle
19 erstes Signal
21 Signalverarbeitungseinrichtung
23 zweites Signal
25 zweite Stelle
27 Drucksensor
29 drittes Signal
31a erster Atemgasfilter
31b zweiter Atemgasfilter
31c dritter Atemgasfilter
33 Mündungsstelle
35a erste Blende
35b zweite Blende
37 Pumpe
39 Absaugeinrichtung
41a erster Druckanschluss
41b zweiter Druckanschluss

**Patentansprüche**

1. Verfahren zum Bestimmen einer Gaskonzentration in einer Hauptleitung (7) eines Beatmungssystems (1), wobei das Beatmungssystem (1) umfasst:

ein Beatmungsgerät (3) mit einem Anschluss (5a) zum Bereitstellen eines Atemgases;
eine Hauptleitung (7) zum Verbinden des Anschlusses (5a) mit einer Patientenschnittstelle (11);
eine Nebenleitung (9), die von der Hauptleitung (7) abzweigt, sodass das Atemgas zumindest teilweise durch die Nebenleitung (9) strömen kann;
einen Gassensor (15) zum Messen einer Gaskonzentration an mindestens einer ersten Stelle (17) in der Nebenleitung (9);
eine Signalverarbeitungseinrichtung (21);
wobei das Verfahren umfasst:

Empfangen eines ersten Signals (19), das die durch den Gassensor (15) gemessene Gaskonzentration anzeigt, in der Signalverarbeitungseinrichtung (21);
Erzeugen eines zweiten Signals (23), das eine geschätzte Gaskonzentration an mindestens einer von der ersten Stelle (17) abweichenden zweiten Stelle (25) in der Hauptleitung (7) anzeigt, aus dem ersten Signal (19) durch die Signalverarbeitungseinrichtung (21), **dadurch gekennzeichnet, dass** die geschätzte Gaskonzentration abhängig von einer zeitlichen Ableitung der gemessenen Gaskonzentration bestimmt wird, wobei ein Zusammenhang zwischen der gemessenen Gaskonzentration und der geschätzten Gaskonzentration durch folgende Gleichung definiert ist:

$$\frac{d}{dt}(ConcSensor) = \frac{1}{\tau}(ConcAirway - ConcSensor),$$

wobei "ConcSensor" für die gemessene Gaskonzentration, "ConcAirway" für die geschätzte Gaskonzentration und $\tau$ für eine Konstante steht, wobei $\tau$ = *VolumeSensor/Flow,* wobei "VolumeSensor" für ein bekanntes Messvolumen des Gassensors (15) und "Flow" für einen bekannten Volumenstrom des Atemgases in der Nebenleitung (9) steht.

2. Verfahren nach Anspruch 1, wobei die mindestens eine zweite Stelle (25) eine Abzweigstelle (13), an der die Nebenleitung (9) von der Hauptleitung (7) abzweigt, umfasst.

3. Verfahren nach einem der vorhergehenden Ansprüche,

wobei das zweite Signal (23) unter Verwendung eines mathematischen Modells des Gassensors (15) erzeugt wird; und/oder
wobei das zweite Signal (23) ferner abhängig von einem bekannten Volumenstrom des Atemgases in der Hauptleitung (7) erzeugt wird.

4. Verfahren nach Anspruch 3,
wobei der bekannte Volumenstrom des Atemgases in der Nebenleitung (9) unabhängig von einem Druck des Atemgases in der Hauptleitung (7) einen konstanten Betrag, insbesondere von 2 ml/s oder weniger, bevorzugt von 1 ml/s oder weniger, und/oder eine konstante Richtung hat.

5. Verfahren nach einem der vorhergehenden Ansprüche,

wobei ferner ein drittes Signal (29), das eine durch einen Drucksensor (27) gemessene Druckdifferenz zwischen verschiedenen Stellen der Hauptleitung (7) anzeigt, in der Signalverarbeitungseinrichtung (21) empfangen wird; wobei das zweite Signal (23) ferner unter Verwendung des dritten Signals (29) erzeugt wird.

6. Signalverarbeitungseinrichtung (21) für ein Beatmungssystem (1), wobei das Beatmungssystem (1) umfasst:

ein Beatmungsgerät (3) mit einem Anschluss (5a) zum Bereitstellen eines Atemgases;
eine Hauptleitung (7) zum Verbinden des Anschlusses (5a) mit einer Patientenschnittstelle (11);
eine Nebenleitung (9), die von der Hauptleitung (7) abzweigt, sodass das Atemgas zumindest teilweise durch die Nebenleitung (9) strömen kann;
einen Gassensor (15) zum Messen einer Gaskonzentration an mindestens einer ersten Stelle (17) in der Nebenleitung (9);
wobei die Signalverarbeitungseinrichtung (21) Mittel umfasst, die konfiguriert sind, um das Verfahren nach einem der vorhergehenden Ansprüche auszuführen.

7. Beatmungssystem (1), umfassend:

ein Beatmungsgerät (3) mit einem Anschluss (5a) zum Bereitstellen eines Atemgases;
eine Hauptleitung (7) zum Verbinden des Anschlusses (5a) mit einer Patientenschnittstelle (11);
eine Nebenleitung (9), die von der Hauptleitung (7) abzweigt, sodass das Atemgas zumindest teilweise durch die Nebenleitung (9) strömen kann;
einen Gassensor (15) zum Messen einer Gaskonzentration an mindestens einer ersten Stelle (17) in der Nebenleitung (9);
eine Signalverarbeitungseinrichtung (21) nach Anspruch 6.

8. Beatmungssystem (1) nach Anspruch 7,
wobei das Beatmungssystem (1) so ausgebildet ist, dass ein Volumenstrom des Atemgases in der Nebenleitung (9) unabhängig von einem Druck des Atemgases in der Hauptleitung (7) einen konstanten Betrag, insbesondere von 2 ml/s oder weniger, bevorzugt von 1 ml/s oder weniger, und/oder eine konstante Richtung hat.

9. Beatmungssystem (1) nach Anspruch 7 oder 8,
wobei die Nebenleitung (9) einen ersten Leitungsabschnitt (9a) und einen zweiten Leitungsabschnitt (9b) umfasst, wobei der Gassensor (15) im ersten Leitungsabschnitt (9a) angeordnet ist und der zweite Leitungsabschnitt (9b) den Gassensor (15) überbrückt.

10. Beatmungssystem (1) nach Anspruch 9,

wobei der erste Leitungsabschnitt (9a) eine erste Blende (35a) zum Einstellen eines Volumenstroms des Atemgases im ersten Leitungsabschnitt (9a) und/oder einen ersten Atemgasfilter (31a) umfasst; und/oder wobei der zweite Leitungsabschnitt (9b) eine zweite Blende (35b) zum Einstellen eines Volumenstroms des Atemgases im zweiten Leitungsabschnitt (9b) und/oder einen zweiten Atemgasfilter (31b) umfasst.

11. Computerprogramm, umfassend Befehle, die einen Prozessor der Signalverarbeitungseinrichtung nach Anspruch 6, beim Ausführen des Computerprogramms durch den Prozessor veranlassen, das Verfahren nach einem der Ansprüche 1 bis 5 auszuführen.

12. Computerlesbares Medium, auf dem das Computerprogramm nach Anspruch 11 gespeichert ist.

**Claims**

1. A method for determining a gas concentration in a main line (7) of a breathing system (1), wherein the breathing system (1) comprises:

   a ventilator (3) with a connection (5a) for providing a respiratory gas;
   a main line (7) for connecting the connection (5a) to a patient interface (11);
   a secondary line (9) that branches off from the main line (7) such that at least some of the respiratory gas can flow through the secondary line (9);
   a gas sensor (15) for measuring a gas concentration at at least one first point (17) in the secondary line (9);
   a signal processing apparatus (21);
   wherein the method comprises:

   receiving a first signal (19), which indicates the gas concentration measured by the gas sensor (15), in the signal processing apparatus (21);
   generating a second signal (23), which indicates an estimated gas concentration at at least one second point (25) in the main line (7), said second point deviating from the first point (17), from the first signal (19) by the signal processing apparatus (21), **characterized in that** the estimated gas concentration is determined depending on a temporal derivation of the measured gas concentration, wherein a relationship between the measured gas concentration and the estimated gas concentration is defined by the following equation:

$$\frac{d}{dt}(ConcSensor) = \frac{1}{\tau}(ConcAirway - ConcSensor),$$

   wherein "ConcSensor" stands for the measured gas concentration, "ConcAirway" stands for the estimated gas concentration, and $\tau$ stands for a constant, wherein $\tau = VolumeSensor/Flow$, wherein "VolumeSensor" stands for a known measured volume of the gas sensor (15) and "Flow" stands for a known volumetric flow rate of the respiratory gas in the secondary line (9).

2. The method according to claim 1, wherein the at least one second point (25) comprises a branching point (13) at which the secondary line (9) branches off from the main line (7).

3. The method according to one of the preceding claims, wherein the second signal (23) is generated using a mathematical model of the gas sensor (15); and/or wherein the second signal (23), furthermore, is generated depending on a known volumetric flow rate of the respiratory gas in the main line (7).

4. The method according to claim 3, wherein the known volumetric flow rate of the respiratory gas in the secondary line (9), regardless of a pressure of the respiratory gas in the main line (7), has a constant quantity, in particular 2 ml/s or less, preferably 1 ml/s or less, and/or has a constant direction.

5. The method according to one of the preceding claims, wherein, furthermore, a third signal (29), which indicates a pressure difference, measured by a pressure sensor (27), between various points in the main line (7), is received in the signal processing apparatus (21); wherein the second signal (23), furthermore, is generated using the third signal (29).

6. A signal processing apparatus (21) for a breathing system (1), wherein the breathing system (1) comprises:

   a ventilator (3) with a connection (5a) for providing a respiratory gas;
   a main line (7) for connecting the connection (5a) to a patient interface (11);
   a secondary line (9) that branches off from the main line (7) such that at least some of the respiratory gas can flow through the secondary line (9);
   a gas sensor (15) for measuring a gas concentration at at least one first point (17) in the secondary line (9);
   wherein the signal processing apparatus (21) comprises means that are configured to perform the method according to one of the preceding claims.

7.  A breathing system (1), comprising:

 a ventilator (3) with a connection (5a) for providing a respiratory gas;
 a main line (7) for connecting the connection (5a) to a patient interface (11);
 a secondary line (9) that branches off from the main line (7) such that at least some of the respiratory gas can flow through the secondary line (9);
 a gas sensor (15) for measuring a gas concentration at at least one first point (17) in the secondary line (9);
 a signal processing apparatus (21) according to claim 6.

8.  The breathing system (1) according to claim 7,
 wherein the breathing system (1) is designed such that a volumetric flow rate of the respiratory gas in the secondary line (9), regardless of a pressure of the respiratory gas in the main line (7), has a constant quantity, in particular 2 ml/s or less, preferably 1 ml/s or less, and/or has a constant direction.

9.  The breathing system (1) according to claim 7 or 8,
 wherein the secondary line (9) comprises a first line portion (9a) and a second line portion (9b), wherein the gas sensor (15) is arranged in the first line portion (9a) and the second line portion (9b) bypasses the gas sensor (15).

10. The breathing system (1) according to claim 9,

 wherein the first line portion (9a) comprises a first flow restrictor (35a) for adjusting a volumetric flow rate of the respiratory gas in the first line portion (9a) and/or a first respiratory gas filter (31a); and/or
 wherein the second line portion (9b) comprises a second flow restrictor (35b) for adjusting a volumetric flow rate of the respiratory gas in the second line portion (9b) and/or a second respiratory gas filter (31b).

11. A computer program, comprising commands which prompt a processor of the signal processing apparatus according to claim 6, upon execution of the computer program by the processor, to perform the method according to one of claims 1 to 5.

12. A computer-readable medium, on which the computer program according to claim 11 is stored.

**Revendications**

1.  Procédé de détermination d'une concentration de gaz dans une conduite principale (7) d'un système respiratoire (1), dans lequel le système respiratoire (1) comporte :

 un appareil respiratoire (3) doté d'un raccord (5a) destiné à fournir un gaz respiratoire ;
 une conduite principale (7) destinée à relier le raccord (5a) à une interface patient (11) ;
 une conduite secondaire (9) bifurquant à partir de la conduite principale (7), de manière à ce que le gaz respiratoire puisse s'écouler au moins partiellement à travers la conduite secondaire (9) ;
 un capteur de gaz (15) destiné à mesurer une concentration de gaz à au moins un premier endroit (17) dans la conduite secondaire (9) ;
 un dispositif de traitement de signal (21) ;
 dans lequel le procédé comporte :

 la réception d'un premier signal (19) indiquant la concentration de gaz mesurée par le capteur de gaz (15), dans le dispositif de traitement de signal (21) ;
 la génération d'un deuxième signal (23) indiquant une concentration de gaz estimée à au moins un deuxième endroit (25) différent du premier endroit (17) dans la conduite principale (7), à partir du premier signal (19), par le dispositif de traitement de signal (21), **caractérisé en ce que** la concentration de gaz estimée est déterminée en fonction d'une dérivée temporelle de la concentration de gaz mesurée, dans lequel un rapport entre la concentration de gaz mesurée et la concentration de gaz estimée est défini par l'équation suivante :

$$\frac{d}{dt}(ConcSensor) = \frac{1}{\tau}(ConcAirway - ConcSensor),$$

où « ConcSensor » représente la concentration de gaz mesurée, « ConcAirway » représente la concentration de gaz estimée et $\tau$ représente une constante, où $\tau = VolumeSensor/Flow$, où « VolumeSensor » représente un volume de mesure connu du capteur de gaz (15) et « Flow » représente un flux volumique connu du gaz respiratoire dans la conduite secondaire (9).

2. Procédé selon la revendication 1,
dans lequel l'au moins un deuxième endroit (25) comporte un point de bifurcation (13) auquel la conduite secondaire (9) bifurque à partir de la conduite principale (7).

3. Procédé selon l'une des revendications précédentes,

dans lequel le deuxième signal (23) est généré à l'aide d'un modèle mathématique du capteur de gaz (15) ; et/ou
dans lequel le deuxième signal (23) est généré en outre en fonction d'un flux volumique connu du gaz respiratoire dans la conduite principale (7).

4. Procédé selon la revendication 3,
dans lequel le flux volumique connu du gaz respiratoire dans la conduite secondaire (9) présente une quantité constante, en particulier de 2 ml/s ou moins, de préférence de 1 ml/s ou moins, et/ou une direction constante, indépendamment d'une pression du gaz respiratoire dans la conduite principale (7).

5. Procédé selon l'une des revendications précédentes,

dans lequel, en outre, un troisième signal (29) indiquant une différence de pression entre différents endroits de la conduite principale (7), mesurée par un capteur de pression (27), est reçu dans le dispositif de traitement de signal (21) ;
dans lequel le deuxième signal (23) est en outre généré à l'aide du troisième signal (29).

6. Dispositif de traitement de signal (21) pour un système respiratoire (1), dans lequel le système respiratoire (1) comporte :

un appareil respiratoire (3) doté d'un raccord (5a) destiné à fournir un gaz respiratoire ;
une conduite principale (7) destinée à relier le raccord (5a) à une interface patient (11) ;
une conduite secondaire (9) bifurquant à partir de la conduite principale (7), de manière à ce que le gaz respiratoire puisse s'écouler au moins partiellement à travers la conduite secondaire (9) ;
un capteur de gaz (15) destiné à mesurer une concentration de gaz à au moins un premier endroit (17) dans la conduite secondaire (9) ;
dans lequel le dispositif de traitement de signal (21) comporte des moyens configurés pour mettre en œuvre le procédé selon l'une des revendications précédentes.

7. Système respiratoire (1), comportant :

un appareil respiratoire (3) doté d'un raccord (5a) destiné à fournir un gaz respiratoire ;
une conduite principale (7) destiné à relier le raccord (5a) à une interface patient (11) ;
une conduite secondaire (9) bifurquant à partir de la conduite principale (7), de manière à ce que le gaz respiratoire puisse s'écouler au moins partiellement à travers la conduite secondaire (9) ;
un capteur de gaz (15) destiné à mesurer une concentration de gaz à au moins un premier endroit (17) dans la conduite secondaire (9) ;
un dispositif de traitement de signal (21) selon la revendication 6.

8. Système respiratoire (1) selon la revendication 7,
dans lequel le système respiratoire (1) est conçu de telle façon qu'un flux volumique du gaz respiratoire dans la conduite secondaire (9) présente une quantité constante, en particulier de 2 ml/s ou moins, de préférence de 1 ml/s ou moins, et/ou une direction constante, indépendamment d'une pression du gaz respiratoire dans la conduite principale (7).

9. Système respiratoire (1) selon la revendication 7 ou 8,
dans lequel la conduite secondaire (9) comporte une première section de conduite (9a) et une deuxième section de conduite (9b), dans lequel le capteur de gaz (15) est disposé dans la première section de conduite (9a) et la deuxième section de conduite (9b) enjambe le capteur de gaz (15).

10. Système respiratoire (1) selon la revendication 9,

dans lequel la première section de conduite (9a) comporte un premier diaphragme (35a) destiné à régler un flux volumique du gaz respiratoire dans la première section de conduite (9a) et/ou un premier filtre à gaz respiratoire (31a) ; et/ou
dans lequel la deuxième section de conduite (9b) comporte un deuxième diaphragme (35b) destiné à régler un flux volumique du gaz respiratoire dans la deuxième section de conduite (9b) et/ou un deuxième filtre à gaz respiratoire (31b).

11. Programme informatique, comportant des ordres amenant un processeur du dispositif de traitement de signal selon la revendication 6 à mettre en œuvre le procédé selon l'une des revendications 1 à 5 lors de l'exécution du programme informatique par le processeur.

12. Support lisible par ordinateur, sur lequel est enregistré le programme informatique selon la revendication 11.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110004108 A1
- US 20210109084 A1
- US 5081871 A
- US 6000397 A